# EUROPÄISCHE PATENTSCHRIFT

(11) **EP 0 928 172 B1**
(45) Veröffentlichungstag und Bekanntmachung des Hinweises auf die Patenterteilung: **06.06.2001**
(21) Anmeldenummer: 98937542.3
(22) Anmeldetag: 24.06.1998
(51) Int. Cl.: A61F 2/38

(54) **STIELLOSE KNIEGELENKENDOPROTHESE**
SHANKLESS KNEE JOINT ENDOPROSTHESIS
PROTHESE D'ARTICULATION DU GENOU SANS TIGE

(30) Priorität: 04.07.1997 DE 19728636; 16.10.1997 DE 19745632
(43) Veröffentlichungstag der Anmeldung: 14.07.1999
(73) Patentinhaber: ESKA Implants GmbH & Co., 23556 Lübeck (DE)
(72) Erfinder: GRUNDEI, Hans, D-23558 Lübeck (DE); THOMAS, Wolfram, I-00135 Roma (IT)
(74) Vertreter: Fuchs Mehler Weiss & Fritzsche
(86) Internationale Anmeldenummer: EP9803857
(87) Internationale Veröffentlichungsnummer: WO9901090

(56) Entgegenhaltungen:
- GB-A- 2 007 980
- US-A- 4 502 161
- US-A- 5 207 711
- US-A- 5 282 867
- US-A- 5 522 902

## Beschreibung

Die vorliegende Erfindung betrifft eine Kniegelenkendoprothese mit einem Femurteil und einem Tibiateil.

Derartige Systeme sind bekannt, beispielsweise aus der DE-A-C-41 41 757.

Darin weist das Femurteil von medial nach lateral gesehen im wesentlichen eine U-Form auf, wobei eine horizontale, zwei diagonale und zwei vertikale femurwärts weisende Anlageflächen vorgesehen sind zur Anlage an einen resezierten Femurknochen. Das Femurteil ist mit zwei Gleitkufen versehen, welche die natürlichen Kondylen und Kondylenrollen nachbilden.

Das Tibiateil bekannter System weist üblicherweise zwei Gleitbahnen auf, auf denen die Gleitkufen des Femurteiles eine Abroll- und gegebenenfalls - je nach Art der Endroprothese - Gleitbewegungen ausführen können. Das Tibiateil weist eine tibiawärts weisende horizontale Auflagefläche zur Auflage auf der resezierten Tibia auf.

Sowohl Tibiateil als auch Femurteil bekannter Systeme weisen jeweils einen konischen Zapfen auf, der in eine konische Klemmhülse in einem jeweiligen Stielteil des modularen Systems steckbar ist. Zwischen Zapfen und Hülse ist eine konische Klemmverbindung herstellbar, die für eine dauerhafte Verbindung zwischen Femurteil und Femurstielteil bzw. Tibiateil und Tibiastielteil sorgen soll.

Die Stiele der modularen Systeme werden entweder mittels eines Knochenzementes wie beispielsweise PMMA in dem Markraum des jeweiligen Röhrenknochens implantiert oder aber zementlos, wobei dann der Stielteil mit einer besonderen Oberfläche versehen ist, in die hinein und durch die hindurch Knochenmaterial zur dauerhaften Sekundärfixation des Implantates wachsen kann (s. bspw. DE-C-195 435 30).

Mit den bekannten Systemen verknüpft sind folgende Probleme:

Die meist aus Metall bestehenden Stiele, die in den Markraum des jeweiligen Röhrenknochens eingesetzt werden, weisen einen völlig anderen Elastizitätsmodul auf als das den Stiel umgebende Knochenmaterial (Spongiosa). Dies führt insbesondere bei älteren Menschen, deren Knochen osteoporotisch befallen sind, oberhalb des Stielendes in dem Markraum oft zu Ermüdungsbrüchen eben auf Grund der unterschiedlichen Elastizitätsmodule.

Das zweite Problem betrifft den natürlichen Valgitätswinkel, der bei den meisten Menschen im Bereich von 7,5° liegt. Mit den künstlichen Endoprothesen-Systemen sind aber bislang nur Valgitätswinkel von maximal 5° darstellbar, und zwar aufgrund der erwähnten, üblicherweise verwendeten konischen Verklemmung zwischen dem Stielteil und dem Femurteil bzw. Tibiateil, da es bei einem Winkel > 5° nicht mehr zu der erwünschten konischen Verklemmung kommt.

Darüber hinaus ist es problematisch bei den bekannten Stielendooprothesen, im Falle eines notwendig werdenden Revisionseingriffes genügend Halt für eine neue Stielendoprothese zu finden, so daß diese hinreichend fest implantiert werden kann. Dies ist insbesondere bei jüngeren Patienten kritisch, da nach bisheriger Erfahrung implantierte Endoprothesen eine begrenzte Lebensdauer von 10 bis 15 Jahren haben, sofern nicht schon vorher ein unvorhergesehener Revisionseingriff nötig wird. Bei jüngeren Patienten ist es dann in jedem Falle erforderlich, die ursprünglich implantierte Endoprothese durch eine Neue zu ersetzen. Dies ist aus erwähnten Gründen nicht ohne weiteres immer möglich.

Die Merkmale des Oberbegriffs des Anspruchs 1 sind aus der GB-A-2007980 bekannt (siehe Figur 15).

Vor diesem Hintergrund ist es nun die Aufgabe der vorliegenden Erfindung, eine völlig neuartige Kniegelenksendoprothese anzugeben, bei der die Probleme der bekannten Endoprothesen hinsichtlich unterschiedlicher Elastizitätsmodule des Implantatmaterials und des umgebenden Knochenmaterials nicht auftreten, und bei der die Einschränkung hinsichtlich des nachahmbaren Valgitätswinkels nicht besteht.

Gelöst wird diese Aufgabe mit bei einer Kniegelenksendoprothese gemäß Anspruch 1. Nachfolgend aufgeführten werden folgende Merkmale aufgeführt:
1. Das Femurteil und das Tibiateil sind stiellos ausgebildet. Dies bedeutet, daß weder das Femurteil noch das Tibiateil mit einem modularen Stielteil zu verbinden sind. Dadurch entfällt die Einschränkung eines Valgitätswinkels auf maximal 5°, da keine konische Klemmverbindung zwischen jeweils einem Stielteil und dem Tibia- bzw. Femurteil hergestellt werden muß. Es entfällt auch die Problematik hinsichtlich der aufeinanderstoßenden unterschiedlichen Elastizitätsmodule im Markraum der Tibia bzw. des Femurs, da kein Stiel in den Markraum eingesetzt wird. Im wesentlichen umgreift also das Femurteil das resezierte Femurende, ohne daß ein Stiel in die Markhöhle des Femurs eindringen würde. Das Tibiateil hingegen liegt auf der resezierten Auflagefläche der Tibia auf, ohne daß es einen der bekannten Stiele aufweist, der über eine konische Klemmverbindung mit ihm gekoppelt werden müßte.
   Hinsichtlich der Valgusstellung sowie hinsichtlich der Vermeidung der aufeinandertreffenden unterschiedlichen E-Module löst das erwähnte Merkmal der Stiellosigkeit die vorerwähnte Aufgabe. Die Langzeitstabilität einer derartig ausgebildeten Endoprothese in situ wir durch das folgende Merkmal erzielt.
2. Mindestens die horizontale und die beiden diagonalen Anlageflächen des Femurteils und die horizontale Auflagefläche des Tibiateils sind mit einer offenmaschigen, dreidimensionalen Raumnetzstruktur versehen, die ein integraler Bestandteil des Grundkörpers des Femurteils und des Tibiateils ist. Letzteres ist besonders hervorzuheben. da die Raumnetzstruktur der einzige Bestandteil der Endoprothese ist, welche für eine stabile Sekundärfixation des Femurteils und des Tibiateils in situ sorgt. Ein etwaig aufgesintertes metallisches Netzwerk würde den Belastungen nicht gerecht werden. Gedacht ist an den Einsatz einer Raumnetzstruktur, wie sie offenbart ist beispielsweise in der DE-C-41 06 971 oder der DE-C-195 43 530, wo sogenannte Tripoden als integraler Bestandteil des Grundkörpers des Femurteils und des Tibiateils in einem feingießtechnischen Verfahren hergestellt werden.
   Damit Knochenmaterial möglichst kurzfristig in die offenmaschige Raumnutzstruktur einorganisiert wird, sind die beiden nachfolgend wiedergegebenen Merkmale vorgesehen:
3. Medial und lateral sind am Femurteil zwei femurwärts angewinkelte Laschen mit jeweils mindestens einer Durchbohrung angeformt, in die jeweils eine Knochenschraube eingesetzt werden kann, und
4. medial und lateral am Tibiateil sind zwei tibiawärts angewinkelte Laschen mit jeweils mindestens einer Durchbohrung angeformt, in die jeweils eine Knochenschraube eingesetzt werden kann. Die Anwinkelung der erwähnten Laschen ist vorliegend von besonderer Bedeutung. Hierdurch nämlich wird beim Einschrauben der jeweiligen Knochenschrauben, die durch die erwähnten Durchbohrungen in den Laschen gesetzt werden, eine Diagonalverspannung vom Femurteil bzw. Tibiateil erzielt, wobei die Schrauben durch das spongiöse Gewebe hin bis zur Gegenkortikalis und darüber hinaus reichen können, so daß sie die Gegenkortikalis sogar durchdringen können. Durch die Diagonalverspannung werden jeweils Kompressionskräfte mit horizontalen und vertikalen Komponenten auf die Schnittstelle zwischen der Raumnetzstruktur und dem resezierten Knochen ausgeübt. Hierdurch wird das Knochenmaterial, welches mit der erwähnten Raumnetzstruktur in Berührung kommt, zum Wachstum angeregt, so daß die Einorganisation in die offenen Maschen beschleunigt vonstatten geht. Die in den Knochen eingeschraubten Schrauben haben daher weniger eine Wirkung im Hinblick auf eine stabile Sekundärfixation, sondern vielmehr im Hinblick auf eine Primärfixation und im Hinblick auf die Ausübung von diagonalwirkenden Kompressionskräften. Nach vollständiger Einorganisation von Knochenmaterial in die Raumnetzstruktur üben die Knochenschrauben keinerlei Funktion mehr aus. Theoretisch ließen sie sich ab diesem Zeitpunkt entfernen, was jedoch in der Praxis nicht ausgeführt wird. da dies eine neue Operation bedeutete.

Mit der beschriebenen anvisierten Sekundärfixation wird zwischen dem künstlichen System der (metallischen) Raumnetzstruktur und dem natürlichen Knochen im wesentlichen eine Osteosynthese nachgebildet.

Das Femurteil und das Tibiateil kann alle bekannten Formen annehmen, angefangen von den Formen einer sogenannten Schlittenendoprothese, wobei die kompletten Seitenbänder und das hintere Kreuzband intakt sein müssen, über jene der sogenannten Gleitachsenendoprothese (DE-A 25 49 819), bei der ein hinteres Kreuzband keine Voraussetzung für eine Implantation ist, die Seitenbänder jedoch noch intakt sind, und weiter über die Form einer sogenannten Kegelendoprothese (DE-A 39 22 294), bei der gegenüber der Gleitachsenendoprothese die Seitenbänder nur noch mäßig intakt sein müssen, bis hin zur Form der sogenannten Polendoprothese (DE-C-41 41 757), bei der auf alle Bänder verzichtet wird.

Der Idealforderung hinsichtlich einer Minimalresektion wird mit der erfindungsgemäßen Endoprothese voll entsprochen. Sollte es dennoch einmal zu einem Revisionseingriff kommen, so lassen sich das Femurteil und das Tibiateil einfach ablösen durch eine Blattsäge, die einfach zwischen das Tibiateil bzw. Femurteil und der Auflage- bzw. den Anlageflächen geführt werden muß, um die sich dort ausgebildeten Knochen-Trapekel zu durchtrennen und die Endoprothesenteile zu entfernen. Hernach besteht immer noch die Möglichkeit der Versorgung des Patienten mit herkömmlichen Stielendoprothesen, wenn die erfindungsgemäße stiellose Endoprothese nicht mehr für eine erneute Implantation in Betracht kommt. Hierbei kann auf den gleichen Typ des künstlichen Kniegelenks zurückgegriffen werden.

Wenn also beispielsweise zunächst eine erfindungsgemäße Endoprothese in Form einer Gleitachsenendoprothese implantiert wird, es jedoch später zu Komplikationen kommt, so ist es unter der Voraussetzung eines gleichen Bänderbildes möglich, eine Stielendoprothese in Ausbildung einer Gleitachsenendoprothese zu implantieren. Entsprechendes gilt für alle anderen erwähnten Prothesentypen.

Somit löst die erfindungsgemäße Endoprothese nicht nur die obenerwähnte Aufgabe. Vielmehr bietet sie auch eine Art von Modularität und Vielseitigkeit im Hinblick auf etwaige Revisionseingriffe, wie es bislang nicht bekannt war.

Weitere vorteilhafte Ausführungsformen sind in den Unteransprüchen angegeben.

Gemäß einer ersten vorteilhaften Weiterbildung ist vorgesehen, daß am Tibiateil ein nach tibiawärts gerichteter Zentrierzapfen angeformt ist. Dieser Zapfen hat mit einem herkömmlichen Stiel nichts gemein. Vielmehr ragt er nur wenige Zentimeter bis Millimeter von der horizontalen Resektionsfläche der Tibia in das Innere des Knochens, um dort die Zentrierung des Tibiateils zu bewerkstelligen. Es ist nämlich von großer Bedeutung, daß das Tibiateil absolut zentriert auf dem Tibiaende sitzt.

Gemäß einer noch weiteren vorteilhaften Ausführungsform ist vorgesehen, daß am Tibiateil tibiawärts ein von medial nach lateral verlaufender Antirotationsschild vorgesehen ist. Dieser Schild dringt in die Spongiosa des Tibiaknochens ein und bietet eine absolute Rotationsstabilität des Tibiateils auf der Tibia.

Vorzugsweise ist der erwähnte Zentrierzapfen - soweit vorhanden - mit einer dreidimensionalen offenmaschigen Raumnetzstruktur belegt, in welche wiederum Knochentrapekel einwachsen können und so für einen dauerhaften Verbund auch des Zapfens mit dem Knochen sorgt.

Vorzugsweise ist vorgesehen, daß der Winkel zwischen den am Tibiateil angewinkelten Laschen und den am Femurteil angewinkelten Laschen zu jeweils der Horizontalen in einem Bereich zwischen 15 und 75° liegt. Besonders bevorzugt wird ein Winkel von 45°, da damit ein ausgewogenes Verhältnis zwischen der Horizontal- und der Vertikalkomponente der eingeleiteten Kompressionskraft gegeben ist.

Keinesfalls sollte die Maschenweite der offenmaschigen Raumnetzstruktur kleiner als 500 µm sein, da ansonsten das Wachstum des Knochens in die Struktur und durch sie hindurch zu gering ausfallen würde, um die ihm zugedachte Funktion der Aufnahme und der Weiterleitung sämtlicher Kräfte, auch bei Lastwechseln, ausüben zu können. Knochenmaterial, das an eine Oberflächenstruktur mit einer Maschenweite von unter 500 µ einwachsen würde, wäre dagegen nur eingeschränkt tragfähig und im Rahmen der vorliegenden Erfindung würde der anvisierte Zweck nicht erreicht werden können.

Bei Diagnosen, bei denen die beiden Seitenbänder sowie das hintere Kreuzband noch weitgehend intakt sind, kann es jedoch zu unphysiologischen Bewegungsabläufen kommen aufgrund der durch die Bänder eingeleiteten

Kräfte.

Diesem Effekt wird entgegengewirkt - also der Bewegungsablauf noch physiologischer nachgebildet - durch eine erfindungsgemäße Weiterbildung, bei der die Gleitbahnen des Tibiateils in einer Kunststoffauflage ausgeformt sind, die um einen in dem Tibiateil eingelassenen Zapfen um einen Winkel α im Bereich α < ± 20° um die Ruhelage schwenkbar oder rotierbar ist.

Ausgehend also von den üblichen Konstellationen eines Tibiateils eines künstlichen Kniegelenks, bei dem nämlich eine Kunststoffauflage starr mit dem meist metallischen übrigen Tibiateil verbunden ist, wird bei dieser Ausführungsform die Auflage um den erwähnten Zapfen herum schwenkbar gehalten. Bevorzugt sitzt der Zapfen, um den die Kunststoffauflage schwenkbar ist, etwa zentrisch im Mittelteil des Tibiateils. Die Hauptachse des Zapfens verläuft von caudal nach kranial.

Diese Weiterbildung bietet die Möglichkeit, daß das Plateau oder die Kunststoffauflage Ausweichbewegungen ausführen kann, wenn etwa die Bänderkräfte bei einer Beugebewegung des künstlichen Kniegelenkes unsymmetrisch sind. In diesem Falle schwenkt das Plateau mit seinen Gleitbahnen in die entsprechende Richtung, so daß die künstlichen Kondylen des Femurteils unter Nachahmung einer physiologischen Bewegung des natürlichen Kniegelenkes auf den Gleitbahnen gleiten und abrollen können.

Gemäß einer noch weiteren bevorzugten Weiterbildung vorerwähnter Ausführungsform ist der Schwenkbereich der Kunststoffauflage begrenzt durch einen an der Peripherie der Auflagefläche des Tibiateils wenigstens teilweise umlaufenden, die Kunststoffauflage mit Spiel einfassenden und als Anschlag für diese wirkenden Rand.

Diese Ausbildung bildet eine hohe Sicherheit gegenüber einer unkontrollierten Rotation der Kunststoffauflage auch unter extremen Bedingungen bzw. Belastungen.

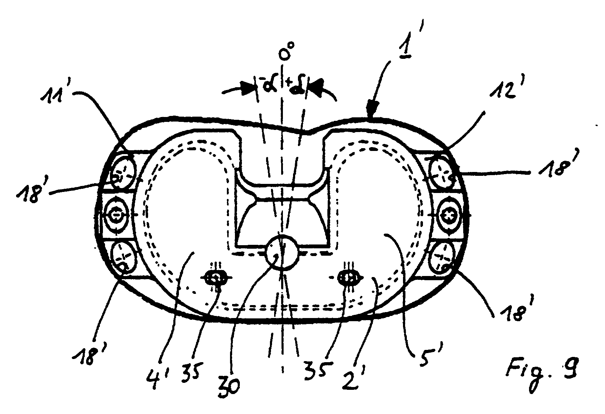

Die Erfindung wird nachfolgend beispielhaft anhand zweier Ausführungsbeispiele näher erläutert wobei die Figuren 9 and 10 keine Ausführungsbeispiele des Erfindung zeigen. Es zeigt:
- Figur 1: die Frontalaufsicht auf ein Tibiateil in Ausbildung der Kniegelenkendoprothese als Schlittenendoprothese,
- Figur 2: einen lateral-medial Schnitt durch das Tibiateil gemäß Figur 1 und einen angedeuteten Tibiaknochen,
- Figur 3: eine Frontalaufsicht auf die Kondylen des Femurteils in Ausbildung der Endoprothese als Schlittenendoprothese,
- Figur 4: eine Schnittansicht entlang der Schnittlinie IV-IV,
- Figur 5: eine Frontalansicht auf das Tibiateil der Kniegelenksendoprothese in Ausbildung als Gleitachsenendoprothese,
- Figur 6: eine Schnittansicht von lateral nach medial durch das Tibiateil gemäß Figur 5 und den Tibiaknochen,
- Figur 7: eine Frontalaufsicht auf das Femurteil in Ausbildung der Kniegelenksendoprothese als Gleitachsenendoprothese,
- Figur 8: eine Schnittansicht entlang der Linie VIII-VIII in Figur 7,
- Figur 9: die Frontalaufsicht auf ein Tibiateil mit schwenkgelagerter Kunststoffauflage, und

Nachfolgend sind gleiche Teile mit denselben Bezugszeichen versehen.

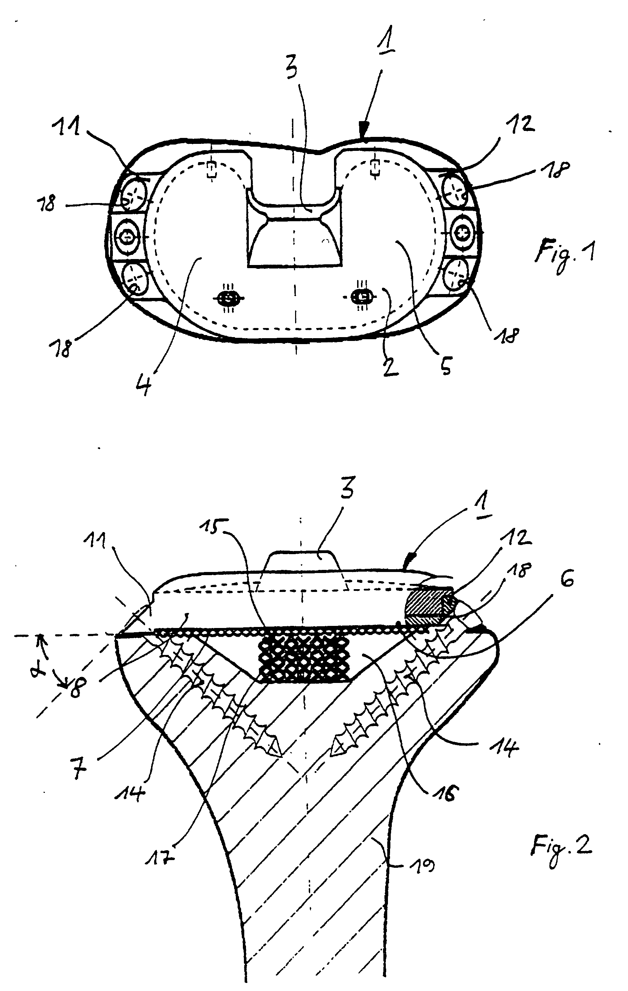

Figur 1 zeigt das Tibiateil 1 in Frontalansicht. Das Inlet 2 weist mittig den Führungssteg 3 auf, der zwischen den Gleitbahnen 4 und 5 ausgebildet ist.

Das Tibiateil 1 weist eine horizontale Auflagefläche 6 (Figur 2) auf, welche plan auf der resezierten Tibiafläche zu liegen kommt. Die Resektionsflächen sowohl an der Tibia 19 als auch am Femur 29 werden vorzugsweise unter Zuhilfenahme der Nagel- und Resektionslehren gemäß der DE 197 16 300 sowie der DE-A-44 23 717 hergestellt.

Die horizontale Auflagefläche 6 des Tibiateils 1 ist tibiawärts mit einer offenmaschigen, dreidimensionalen Raumnetzstruktur 7 versehen, in welche hinein und durch welche hindurch Knochentrapekel zur Sekundärfixation des Tibiateils 1 an der Tibia 19 wachsen sollen. Die Raumnetzstruktur 7 ist ein integraler Bestandteil des Grundkörpers 8 des Tibiateils 1. Vorzugsweise ist der Grundkörper 8 zusammen mit der Raumnetzstruktur 7 in einem feingießtechnischen Verfahren in einem Zuge hergestellt. Dies gewährleistet die notwendige Festigkeit der Struktur am Grundkörper 8.

Jeweils eine tibiawärts im Winkel α zur Horizontalen angewinkelte Lasche 11 und 12 ist lateral bzw. medial am Tibiateil 1 angeformt. Die Laschen 11 und 12 sind mit mindestens einer Durchbohrung 18 versehen, vorliegend jeweils mit drei Durchbohrungen. Durch diese Durchbohrung 18 kann eine Knochenschraube 14 gesetzt werden und entsprechend der Winkelneigung α der Laschen 11 und 12 schräg in den Tibiaknochen geschraubt werden. Hierdurch wird die erwünschte Spannung des Tibiateils 1 auf der Tibia 19 erzeugt. Die erzeugte Diagonalspannung führt dazu, daß die Raumnetzstruktur 7 in Figur 2 nach unten gedrückt wird, so daß das darunterliegende Knochenmaterial zum Einsprossen in die Raumnetzstruktur 7 angeregt wird.

Vorliegend weist das Tibiateil 1 noch einen zur Tibia 19 hin angesetzten Zentrierzapfen 15 auf, der vorliegend ebenfalls mit einer dreidimensionalen offenmaschigen Raumnetzstruktur 17 belegt ist.

Der Zentrierzapfen 15 sorgt für eine optimale Lage des Tibiateils 1 auf dem resezierten Tibiastumpf. Die optimale Lage wird stabil gehalten vorliegend durch einen angeformten Antirotationsschild 16, der von medial nach lateral verläuft und den Zentrierzapfen 15 einfaßt.

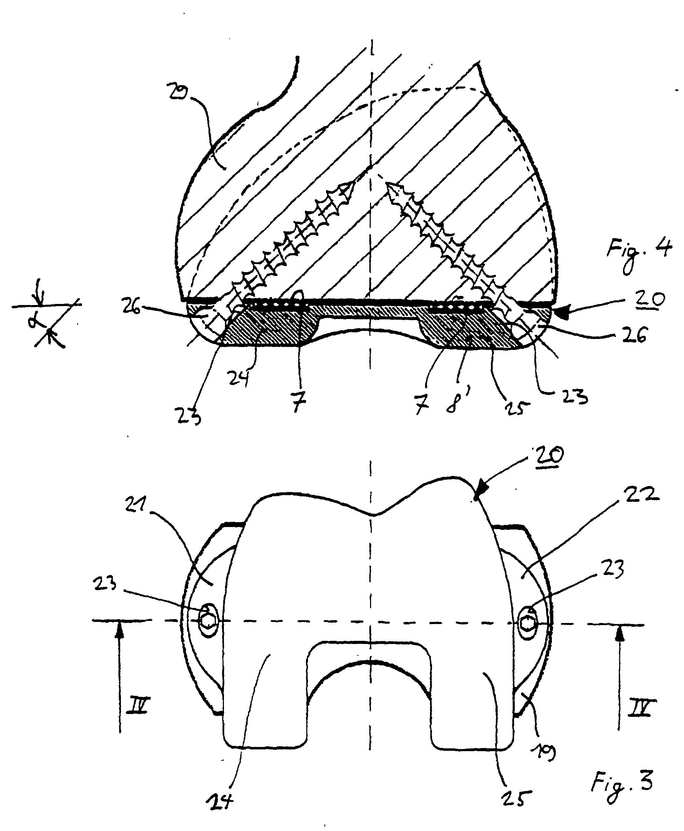

Der Figur 3 ist ein Femurteil 20 zu entnehmen, welches mit dem Tibiateil 1 aus Figur 1 eine Schlittenendoprothese bildet. Das Femurteil 20 verfügt über zwei den natürlichen Kondylen nachempfundenen Gleitkufen 24 und 25. Lateral und medial angeformt ist jeweils eine femurwärts angewinkelte Lasche 21, 22 mit einer Durchbohrung 23. Die Implantationslage ergibt sich aus Figur 4.

Vorliegend ist lediglich die horizontale Anlagefläche des Femurteils 20 erkennbar, und zwar wie sie belegt ist mit der Raumnetzstruktur 7. Ebenfalls mit dieser Raumnetzstniktur 7 belegt sind die diagonalen Anlageflächen (nicht dargestellt).

Durch die Durchbohrungen 23 ist jeweils eine Knochenschraube 26 gesetzt und in den Knochen des Femurs 29 geschraubt. Die Anwinkelung der Laschen 21 und 22 unter einem Winkel α zur Horizontalen sorgt wie schon im Fall des Tibiateils 1 für eine Diagonalverspannung des Femurteils 20 am Femurknochen 29 und damit für eine Kompression der Resektionsflächen am Femur 29 durch das Femurteil 20, wodurch wiederum der Knochen zu einem verstärkten Wachstum in die Raumnetzstruktur 7 hinein und durch sie hindurch angeregt wird.

Damit die Verwachsung des Femurteils 20 mit dem Femurknochen 29 dauerhaft ist, ist die Raumnetzstruktur 7 als integraler Bestandteil des Grundkörpers 8' des Femurteils 20 ausgebildet.

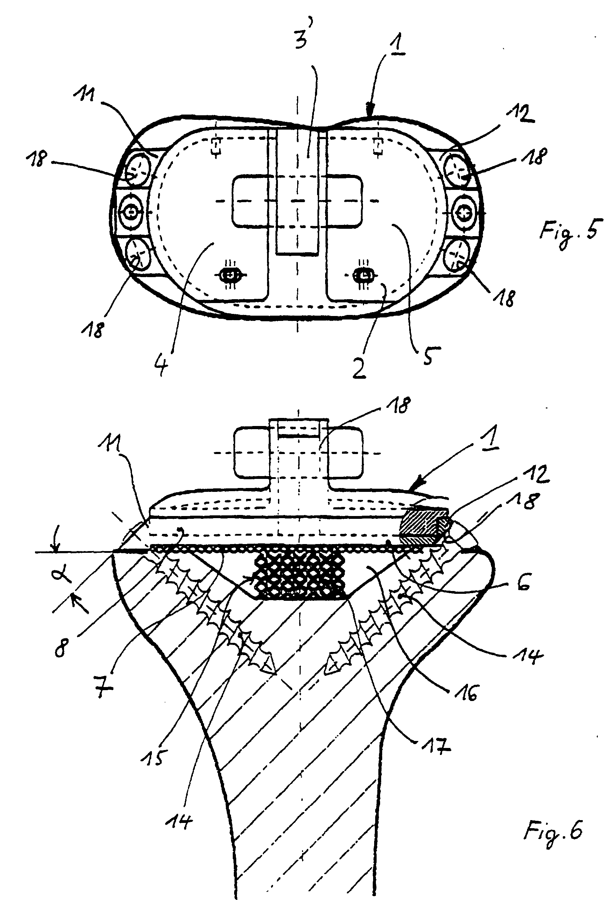

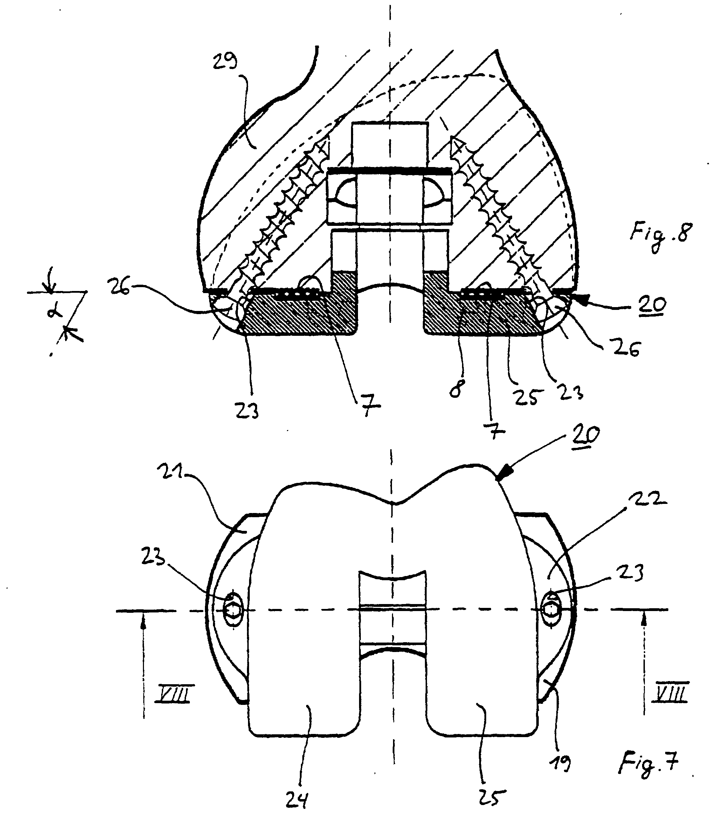

Die Figuren 5 bis 8 zeigen eine weitere Ausführungsform einer Kniegelenksendoprothese, und zwar in Ausbildung als Gleitachsenendoprothese, wie sie sich beispielsweise aus der DE 25 49 819 ergibt. Diese Darstellungen dienen der Veranschaulichung der Tatsache, daß das Femurteil 20 und das Tibiateil 1 alle bekannten Formen von Kniegelenksendoprothesen annehmen können, das System also überaus vielseitig ist. Unterschiede zu der Ausführungsform gemäß den Figuren 1 bis 4 bestehen hinsichtlich der Ausbildung und des Zusammenspiels des Femurteils 20 und des Tibiateils 1. Die erfindungswesentlichen Merkmale jedoch sind wiederum die angewinkelten Seitenlaschen 11,12 und 21,22 sowie die Raumnetzstruktur 7 an den Auflageflächen bzw. Auflagefläche.

Die Figur 9 zeigt eine bevorzugte Weiterbildung der Endorpothese, die insbesondere bei nach weitgehend intakten Seitenbändern sowie intaktem Kreuzband zum Einsatz kommt.

In der Kunststoffauflage 2' sind die beiden Gleitbahnen 4' und 5' ausgeformt. Im Zentrum der Kunststoffauflage 2' ist diese um einen in dem Tibiateil 1' eingelassenen Zapfen 30 schwenkbar, und zwar um einen Winkel α im Bereich zwischen -20 und +20° von der zentrischen Ruhelage aus gesehen. Hierdurch werden Ausweichbewegungen der Kunststoffauflage 2' möglich aufgrund unsymmetrisch wirkender Kräfte in den weitgehend intakten Seitenbändern und hinterem Kreuzband. Die Ausweichbewegung führt dazu, daß die Kondylen des Femurteiles stets auf einer maximalen Gleit- und Abrollfläche abrollen können. Hierdurch wird ein nahezu optimaler physiologischer Bewegungsablauf ermöglicht.

Der Schwenkbereich der Kunststoffauflage 2' ist vorliegend begrenzt durch einen an der Peripherie der Auflagefläche 31 des Tibiateils 1' wenigstens teilweise umlaufenden Rand 32. Der Rand 32 faßt die Kunststoffauflage 2' mit Spiel ein und wirkt als Anschlag für sie. Unter anderem die Größe des Spiels definiert letztendlich den Schwenkbereich der Kunststoffauflage 2'.

Durchbohrungen 35 in der Kunststoffauflage 2' gestatten die Umspülung des gesamten Gelenkes mit der Gelenkflüssigkeit, der sogenannten Synovia.

## Patentansprüche

1. Kniegelenkendoprothese mit
- einem Femurteil (20) welches tibiawärts mit zwei Gleitkufen (24,25) versehen ist, und
- einem Tibiateil (1), welches zwei Gleitbahnen (4,5) aufweist, auf denen die Gleitkufen (24,25) des Femurteiles (20) eine Abroll- und ggf. Gleitbewegung ausführen können, mit einer tibiawärts weisenden horizontalen Auflagefläche (6) zur Auflage auf der resezierten Tibia (19), wobei
- daß das Femurteil (20) und das Tibiateil (1) stiellos ausgebildet sind, und wobei
- medial und lateral am Femurteil (20) zwei femurwärts angewinkelte Laschen (21,22) mit jeweils mindestens einer Durchbohrung (23) angeformt sind, in die jeweils eine Knochenschraube (26) eingesetzt werden kann,
**dadurch gekennzeichnet,**
- daß das Femurteil (20) von medial nach lateral gesehen im wesentlichen U-förmigen mit einer horizontalen, zwei diagonalen und zwei vertikalen, femurwärts weisenden Anlageflächen zur Anlage an einen resezierten Femurknochen (29) ausgebildet ist,
- daß zumindest die horizontale und die beiden diagonalen Anlageflächen des Femurteils (20) und die horizontale Auflagefläche (6) des Tibiateils (1) mit einer offenmaschigen, dreidimensionalen Raumnetzstruktur (7) versehen sind, die ein integraler Bestandteil des Grundkörpers (8,8') des Femurteils (20) und des Tibiateils (1) ist, und
- daß medial und lateral am Tibiateil (1) zwei tibiawärts angewinkelte Laschen (11,12) mit jeweils mindestens einer Durchbohrung (18) angeformt sind, in die jeweils eine Knochenschraube (14) eingesetzt werden kann.

2. Kniegelenkendroprothese nach Anspruch 1, bei der am Tibiateil (1) tibiawärts ein Zentrierzapfen (15) angeformt ist.

3. Kniegelenkendroprothese nach Anspruch 1 oder 2, bei der am Tibiateil (1) tibiawärts ein von medial nach lateral verlaufender Antirotationsschild (16) vorgesehen ist.

4. Kniegelenkendroprothese nach Anspruch 2, bei der der Zentrierzapfen eine dreidimensionale offenmaschige Raumnetzstruktur (17) aufweist.

5. Kniegelenkendroprothese nach einem der Ansprüche 1 bis 4, bei der der Winkel α zwischen den am Tibiateil (1) und am Femurteil (20) angewinkelten Laschen (11,12:21,22) und der Horizontalen in einem Bereich von 15° < α < 75° liegt.

6. Kniegelenkendroprothese nach Anspruch 5, bei der Winkel α = 45° beträgt.

7. Kniegelenkendroprothese nach einem der Ansprüche 1 bis 5, bei der die Maschenweite der offenmaschigen Raumnetzstruktur (7) im Minimum 500 µ beträgt.

8. Kniegelenkendoprothese nach einem der Ansprüche 1 bis 7, bei der die Gleitbahnen (4', 5') des Tibiateils (1') in einer Kunststoffauflage (2') ausgeformt sind, die um einen in dem Tibiateil (1') eingelassenen Zapfen (30) um einen Winkel α im Bereich α< ± 20° um die Ruhelage schwenkbar ist.

9. Kniegelenkendoprothese nach Anspruch 8, bei der der Schwenkbereich der Kunststoffauflage (2') begrenzt ist durch einen an der Peripherie der Auflagefläche (31) des Tibiateils (1') wenigstens teilweise umlaufenden, die Kunststoffauflage (2') mit Spiel einfassenden und als Anschlag wirkenden Rand (32).

## Claims

1. Knee joint endoprosthesis having a femur part (20), essentially U-shaped seen medially to laterally having one horizontal, two diagonal and two vertical contact surfaces pointing towards the femur for resting against a resected femur bone (29), which is provided with two runners (24, 25) in the direction of the tibia, and a tibia part (1), which has two slide tracks (4, 5), on which the runners (24, 25) of the femur part (20) may execute a rolling-off movement and optionally a sliding movement, having a horizontal support surface (6) pointing in the direction of the tibia for resting on the resected tibia (19), wherein the femur part (20) and the tibia part (1) are designed without stems, and wherein two cover plates (21, 22) bent in the direction of the femur each having at least one bore (23) are formed medially and laterally on the femur part (20), and into which in each case one bone screw (26) may be inserted, characterised in that the femur part (20) is designed essentially U-shaped seen medially to laterally having one horizontal, two diagonal and two vertical contact surfaces pointing towards the femur for resting against a resected femur bone (29), in that at least the horizontal and the two diagonal contact surfaces of the femur part (20) and the horizontal support surface (6) of the tibia part (1) are provided with an open-mesh, three-dimensional spatial network structure (7), which is an integral component of the base structure (8, 8') of the femur part (20) and of the tibia part (1), and in that two cover plates (11, 12) bent in the direction of the tibia each having at least one bore (18) are formed medially and laterally on the tibia part (1), and into which in each case one bone screw (14) may be inserted.

2. Knee joint endoprosthesis according to claim 1, in which a centering pin (15) is formed on the tibia part (1) in the direction of the tibia.

3. Knee joint endoprosthesis according to claim 1 or 2, in which an antirotation shield (16) running medially to laterally is provided on the tibia part (1) in the direction of the tibia.

4. Knee joint endoprosthesis according to claim 2, in which the centering pin has a three-dimensional open-mesh spatial network structure (17).

5. Knee joint endoprosthesis according to one of claims 1 to 4, in which the angle @ between the cover plates (11, 12; 21,22) bent on the tibia part (1) and on the femur part (20) and the horizontal lies in a range from 15° < @ < 75°.

6. Knee joint endoprosthesis according to claim 5, in which angle @ = 45°.

7. Knee joint endoprosthesis according to one of claims 1 to 5, in which the mesh width of the open-mesh spatial network structure (7) is 500 µ minimum.

8. Knee joint endoprosthesis according to one of claims 1 to 7, in which the slide tracks (4', 5') of the tibia part (1') are made in a plastic support (2'), which can be pivoted about a pin (30) embedded in the tibia part (1') about an angle @ in the range @ < ± 20° about the rest position.

9. Knee joint endoprosthesis according to claim 8, in which the pivoting range of the plastic support (2') is defined by an edge (32) at least partly running around the periphery of the support surface (31) of the tibia part (1'), enclosing the plastic support (2') with clearance and acting as a stop.

## Revendications

1. Endoprothèse d'articulation du genou avec
- une partie fémorale (20) munie en direction du tibia de deux patins de glissement (24, 25) et
- une partie tibiale (1) présentant deux plages de glissement (4, 5) sur lesquelles les patins de glissement (24, 25) de la partie fémorale (20) peuvent effectuer un mouvement de roulement et le cas échéant de glissement, avec une surface d'appui horizontale (6) dirigée vers le tibia pour venir en appui sur le tibia réséqué (19), dans laquelle
- la partie fémorale (20) et la partie tibiale (1) sont réalisées sans tiges, et dans laquelle
- deux pattes (21, 22) faisant un angle en direction du fémur sont formées d'un seul tenant en position médiale et en position latérale sur la partie fémorale (20), avec chacune au moins un perçage (23) dans lequel une vis à os (26) peut être introduite,
caractérisée en ce que
- la partie fémorale (20) vue dans le sens médial vers latéral a sensiblement une forme de U avec des surfaces d'appui, une horizontale, deux diagonales et deux verticales, dirigées vers le fémur pour venir en appui sur un os fémoral réséqué (29),
- au moins la surface d'appui horizontale et les deux surfaces d'appui diagonales de la partie fémorale (20) et la surface d'appui horizontale (6) de la partie tibiale (1) sont munies d'une structure déployée en trois dimensions à mailles ouvertes (7) qui fait partie intégrante de l'élément de base (8, 8') de la partie fémorale (20) et de la partie tibiale (1), et
- deux pattes (11, 12) faisant un angle en direction du tibia sont formées d'un seul tenant en position médiale et en position latérale sur la partie tibiale (1), avec chacune au moins un perçage (18) dans lequel une vis à os (14) peut être introduite.

2. Endoprothèse d'articulation du genou selon la revendication 1, dans laquelle un téton de centrage (15) dirigé vers le tibia est formé d'un seul tenant sur la partie tibiale (1).

3. Endoprothèse d'articulation du genou selon la revendication 1 ou la revendication 2, dans laquelle est prévu, sur la partie tibiale (1), un plastron anti-rotation (16) dirigé vers le tibia et s'étendant dans le sens médial vers latéral.

4. Endoprothèse d'articulation du genou selon la revendication 2, dans laquelle le téton de centrage présente une structure déployée (17) à mailles ouvertes en trois dimensions.

5. Endoprothèse d'articulation du genou selon l'une des revendications 1 à 4, dans laquelle l'angle α entre les pattes (11, 12 ; 21, 22) faisant un angle avec la partie tibiale (1) et avec la partie fémorale (20) et l'horizontale se situe dans la plage de 15° < α < 75°.

6. Endoprothèse d'articulation du genou selon la revendication 5, dans laquelle l'angle α vaut 45°.

7. Endoprothèse d'articulation du genou selon l'une des revendications 1 à 5, dans laquelle la taille de mailles de la structure déployée à mailles ouvertes (7) est au minimum de 500 µm.

8. Endoprothèse d'articulation du genou selon l'une des revendications 1 à 7, dans laquelle les plages de glissement (4', 5') de la partie tibiale (1') sont formées dans une garniture en matière plastique (2') qui peut pivoter autour d'un téton (30) inclus dans la partie tibiale (1') suivant un angle α dans la plage α < ± 20° de part et d'autre de la position de repos.

9. Endoprothèse d'articulation du genou selon la revendication 8, dans laquelle la plage de pivotement de la garniture en matière plastique (2') est limitée par un rebord (32) qui entoure au moins en partie la périphérie de la surface d'appui (31) de la partie tibiale (1'), qui renferme la garniture en plastique (2') avec du jeu et qui lui sert de butée.
